(19) Europäisches Patentamt
European Patent Office
Office européen des brevets

(11) **EP 4 643 842 A1**

(12) **EUROPEAN PATENT APPLICATION**

(43) Date of publication:
**05.11.2025 Bulletin 2025/45**

(21) Application number: **24181862.4**

(22) Date of filing: **13.06.2024**

(51) International Patent Classification (IPC):
**A61K 6/54** (2020.01)   **A61K 6/56** (2020.01)
**A61K 6/849** (2020.01)   **A61K 6/802** (2020.01)
**A61K 6/818** (2020.01)   **C01B 33/24** (2006.01)
**A61K 6/853** (2020.01)

(52) Cooperative Patent Classification (CPC):
**A61K 6/54; A61K 6/56; A61K 6/802; A61K 6/818;
A61K 6/849; A61K 6/853; C01B 33/24**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.04.2024 CN 202410531845**

(71) Applicant: **Guilin Kevin Peter Technology Co.,
Ltd.**
**Guilin, Guangxi 541100 (CN)**

(72) Inventors:
• **Pang, Xinkuan**
  **Guilin 541100 (CN)**
• **Wu, Changhong**
  **Guilin 541100 (CN)**

(74) Representative: **Zenz Patentanwälte
Partnerschaft mbB
Gutenbergstraße 39
45128 Essen (DE)**

(54) **STRONTIUM-DOPED ROOT CANAL FILLING PASTE, PREPARATION METHOD THEREFOR AND USE THEREOF**

(57)   The present application relates to the technical field of medical materials, and specifically to a strontium-doped root canal filling paste, a preparation method therefor and use thereof. Raw materials of the strontium-doped root canal filling paste include a component (a) and a component (b), wherein the component (a) is selected from calcium silicate compounds, the component (b) is selected from strontium compounds, the component (a) accounts for 20%-40% of a total mass of the raw materials, the component (b) accounts for 0.5%-10% of the total mass of the raw materials; and the component (a) and the component (b) are doped in a physical doping manner. The strontium-doped root canal filling paste can simultaneously address the problems of long curing time, low strength, bad mineralization property and insufficient effect of bonding with a root canal wall of the existing strontium-doped root canal filling paste.

**FIG. 2**

# EP 4 643 842 A1

**Description**

## TECHNICAL FIELD

The present application relates to the technical field of medical materials, and specifically to a strontium-doped root canal filling paste, a preparation method therefor and use thereof.

## BACKGROUND ART

Mineral trioxide aggregate (MTA), commonly known as root canal cement, is a new calcium silicate-based root canal filling material. In 1998, Mahmoud Torabinejad disclosed a method of repairing a root canal with MTA material for the first time (U.S. patent No. 5,769,638). MTA ingredients, similar to those of Ordinary Portland Cement (OPC), mainly include tricalcium silicate (chemical formula CasSiOs, abbreviated as $C_3S$), dicalcium silicate (chemical formula $Ca_2SiO_4$, abbreviated as $C_2S$), and tricalcium aluminate. The MTA is hydrated when mixed with water, and is solidified and hardened through precipitation of colloidal calcium silicate hydrate (C-S-H). The advantages of the MTA as the root canal filling material lie in better biocompatibility and antibacterial property, no shrinkage in curing, and better sealing property. Besides, the MTA is free from the influence of the presence of moisture or blood in curing, has high compressive strength after the curing, and is considered as an optimal root canal filling material at present.

Mixing needs to be performed before use of the conventional MTA, which is not only troublesome but also wasteful. This is improved in the prior art, for example, premixing a calcium silicate compound with an organic solvent to form a paste, while there are many problems with the paste, for example, (1) long setting time and a long treatment period caused by the long setting time; (2) insufficient mineralization property; and (3) poor bonding strength with a root canal wall.

The inventors partially substituted Ca atoms in calcium silicate molecules with Sr atoms to incorporate Sr element into calcium silicate lattices, so as to form a single substance, that is, the inventors chemically doped strontium into the calcium silicate compound to improve the paste (CN113304056B). Although this improvement ameliorates some properties of the paste, comprehensive properties of the paste, such as overall mineralization property, effect of bonding with the root canal wall and setting time, cannot achieve desired effects.

In view of this, the present application is specifically proposed.

## SUMMARY

The present application aims at providing a strontium-doped root canal filling paste, a preparation method therefor and use thereof. The strontium-doped root canal filling paste provided in the embodiments of the present application can simultaneously address the problems of defects of the existing strontium-doped root canal filling paste, such as long curing time, low strength, bad mineralization property and insufficient effect of bonding with a root canal wall.

The present application is implemented as follows.

In the first aspect, the present application provides a strontium-doped root canal filling paste, wherein raw materials of the strontium-doped root canal filling paste include a component (a) and a component (b), and wherein the component (a) is selected from calcium silicate compounds, the component (b) is selected from strontium compounds, the component (a) accounts for 20%-40% of a total mass of the raw materials, the component (b) accounts for 0.5%-10% of the total mass of the raw materials; and the component (a) and the component (b) are doped in a physical doping manner.

In an optional embodiment, the component (a) accounts for 25%-35% of the total mass of the raw materials.

Preferably, the component (a) accounts for 2-5% of the total mass of the raw materials.

In an optional embodiment, the calcium silicate compounds are any one or a combination of any two or more selected from the group consisting of tricalcium silicate, dicalcium silicate and monocalcium silicate.

Preferably, the strontium compound is any one or a combination of any two or more selected from the group consisting of strontium chloride, strontium phosphate, strontium nitrate, strontium fluoride, strontium acetate and strontium ranelate.

In an optional embodiment, the raw materials further include a component (c), and the component (c) is selected from radiation inhibitors.

Preferably, the radiation inhibitors are selected from the group consisting of metal oxides, metal sulfate compounds and polyhalogenated C1-C3 alkyl.

Preferably, the radiation inhibitors are selected from the group consisting of transition metal oxides, metal sulfate compounds and polyiodine-substituted C1-C3-alkyl.

Preferably, the radiation inhibitors are any one or a combination of any two or more selected from the group consisting of niobium oxides, zirconium oxides, bismuth oxides, tantalum oxides, barium sulfate and iodoform.

Preferably, the component (c) accounts for 30%-40% of the total mass of the raw materials, preferably 30%-35%.

In an optional embodiment, the raw materials further include a component (d), and the component (d) is selected from calcium phosphate compounds, preferably, any one or a combination of any two or more selected from the group consisting of α-tricalcium phosphate, β-tricalcium phosphate, tetracalcium phosphate, calcium dihydrogen phosphate and hydroxyapatite.

Preferably, the component (d) accounts for 2%-10% of the total mass of the raw materials, preferably 3%-5%.

In an optional embodiment, the raw materials further include a component (e), and the component (e) is selected from organic solvents which can be mixed with the component (a) and the component (b) to form a paste.

Preferably, the organic solvents are any one or a combination of any two or more selected from the group consisting of alcohol solvents, polymer solvents and sulfoxide solvents.

Preferably, the organic solvents are selected from the group consisting of C2-C5 polyol, poly C2-C5 polyol, polypyrrolidine compounds and C2-C5 sulfoxide solvents.

Preferably, the organic solvents are any one or a combination of any two or more selected from the group consisting of polyethylene glycol, propylene glycol, glycerol, polyvinylpyrrolidone and dimethyl sulfoxide.

Preferably, the component (e) accounts for 20%-40% of the total mass of the raw materials.

In the second aspect, the present application provides use of the strontium-doped root canal filling paste according to any one of the preceding embodiments in preparation of a material for treatment of dental pulp disease.

In the third aspect, the present application provides a preparation method for the strontium-doped root canal filling paste of any one of the preceding embodiments, including: physically mixing the component (a) and the component (b) uniformly.

In an optional embodiment, the preparation method includes: ball-milling and mixing the component (a) and the component (b) to form a mixed powder; and then mixing the mixed powder with at least one of a component (c), a component (d) and a component (e) uniformly.

The present application has the following beneficial effects: for the strontium-doped root canal filling paste provided in the examples of the present application, the calcium silicate compound and the strontium compound are doped in a physical doping manner, which improves mineralization property and biological activity of the strontium-doped root canal filling paste, can greatly shorten setting time of the strontium-doped root canal filling paste, and improve its bondability with the root canal wall, so that the root canal is better sealed.

## BRIEF DESCRIPTION OF DRAWINGS

In order to more clearly illustrate technical solutions of examples of the present application, drawings which need to be used in the examples will be introduced briefly below. It should be understood that the drawings merely show some examples of the present application, and thus should not be considered as limitation to the scope. Those ordinarily skilled in the art still could obtain other relevant drawings according to these drawings, without using any inventive efforts.

FIG. 1 shows a syringe containing a strontium-doped root canal filling paste provided in examples of the present application; and
FIG. 2 shows detection results provided in Detection Example 2 of the present application.

## DETAILED DESCRIPTION OF EMBODIMENTS

In order to make the objectives, technical solutions and advantages of the examples in the present application clearer, the technical solutions in the examples of the present application will be described clearly and completely below. If no specific conditions are specified in the examples, they are carried out under normal conditions or conditions recommended by the manufacturer. If the manufacturers of reagents or apparatus used are not specified, they are conventional products commercially available.

The inventors modified the existing strontium-doped root canal filling paste before by partially substituting Ca atoms in calcium silicate molecules with Sr atoms, to incorporate Sr element into calcium silicate lattices, so as to form a single substance. However, the inventors found that chemically doping strontium into a calcium silicate compound only can improve some properties of the strontium-doped root canal filling paste, but mineralization property, effect of bonding with a root canal wall and setting time of the strontium-doped root canal filling paste still cannot be improved at the same time. The inventors further analyzed the strontium-doped root canal filling paste previously improved, for example, explored and studied selection of the doping atoms Sr, a doping method of Sr, a doping amount of Sr, and selection of calcium silicate, and found that doped elements were hard to release after the chemical doping of Sr, and subsequently even if the paste is improved with Sr, the properties of the strontium-doped root canal filling paste still cannot be improved comprehensively. Based on this, the examples of the present application provide a strontium-doped root canal filling paste, of which raw materials include a component (a) and a component (b), wherein the component (a) is selected from calcium silicate compounds, the component (b) is selected from strontium compounds, and the component (a) and the component (b) are doped in a physical doping manner.

The physical doping of the calcium silicate compound and strontium compound can facilitate the Sr element in exerting its efficacy, and can simultaneously improve the formed strontium-doped root canal filling paste in mineralization property, biological activity, setting time, bondability to the root canal wall and so on.

Specifically, the component (a) accounts for 20%-40% of a total mass of the raw materials, for example, any numerical value within 20%-40%, such as 20%, 25%, 30%, 35% and 40%, or a range value between any two numerical values, preferably, for example, 25-35%.

The component (b) accounts for 0.5%-10% of the total mass of the raw materials, for example, any numerical value within 0.5%-10%, such as 0.5%, 1%, 2%, 5%, 7% and 10%, or a range value between any two numerical values, preferably, for example, 2%-5%.

The component (a) and the component (b) in the above contents can further ensure the properties of the formed strontium-doped root canal filling paste.

Further, the calcium silicate compounds are any one or a combination of any two or more selected from the group consisting of tricalcium silicate, dicalcium silicate and monocalcium silicate; the strontium compound is any one or a combination of any two or more selected from the group consisting of strontium chloride, strontium phosphate, strontium nitrate, strontium fluoride, strontium acetate and strontium ranelate.

The raw materials further include a component (c), wherein the component (c) is selected from radiation inhibitors, and the radiation inhibitors are selected from the group consisting of metal oxides, metal sulfate compounds and polyhalogenated C1-C3 alkyl, preferably, transition metal oxides, metal sulfate compounds and polyiodine-substituted C1-C3-alkyl, for example, any one or a combination of any two or more of niobium oxides, zirconium oxides, bismuth oxides, tantalum oxides, barium sulfate and iodoform.

Further, the component (c) accounts for 30%-40% of the total mass of the raw materials, for example, any numerical value within 30%-40%, such as 30%, 31%, 32%, 33%, 34%, 35%, 36%, 37%, 38%, 39% and 40%, or a range value between any two numerical values, preferably, for example, 30%-35%.

The raw materials further include a component (d), wherein the component (d) is selected from calcium phosphate compounds, for example, including but not limited to any one or a combination of any two or more selected from the group consisting of $\alpha$-tricalcium phosphate, $\beta$-tricalcium phosphate, tetracalcium phosphate, calcium dihydrogen phosphate and hydroxyapatite.

Further, the component (d) accounts for 2%-10% of the total mass of the raw materials, for example, any numerical value within 2-10%, such as 2%, 3%, 4%, 5%, 6%, 7%, 8%, 9% and 10%, or a range value between any two numerical values, preferably, for example, 3%-5%.

Further, the raw materials further include a component (e), wherein the component (e) is selected from organic solvents which can be mixed with the component (a) and the component (b) to form a paste, and wherein the organic solvents are any one or a combination of any two or more selected from the group consisting of alcohol solvents, polymer solvents and sulfoxide solvents, preferably C2-C5 polyol, poly C2-C5 polyol, polypyrrolidine compounds and C2-C5 sulfoxide solvents; for example, the organic solvents include, but not limited to, any one or a combination of any two or more of polyethylene glycol, propylene glycol, glycerol, polyvinylpyrrolidone and dimethyl sulfoxide.

The component (e) accounts for 20%-40% of the total mass of the raw materials, for example, any numerical value within 20%-40%, such as 20%, 25%, 30%, 35% and 40%, or a range value between any two numerical values.

In the second aspect, the present application provides use of the strontium-doped root canal filling paste of any one of the preceding embodiments in preparation of a material for treatment of dental pulp disease.

In the third aspect, the present application provides a preparation method for the strontium-doped root canal filling paste of any one of the preceding embodiments, including: physically mixing the component (a) and the component (b) uniformly.

Specifically, the component (a) and the component (b) are ball-milled to form a mixed powder, wherein a media-to-material ratio (ball-to-material ratio) of a ball mill is (5-15) : 1, a rotational speed is 200 r/min-400 r/min, and then the mixed powder is added, together with at least one of the component (c), the component (d) and the component (e), to a mixer and mixed uniformly, so as to obtain the strontium-doped root canal filling paste. When needed, the strontium-doped root canal filling paste is dispensed into a medical syringe fitted with an injection hose needle, and it is ready for use.

The characteristics and performances of the present application are further described in detail below in conjunction with examples.

Example 1 - Example 16

Example 1 - Example 16 respectively provide a strontium-doped root canal filling paste, of which ingredients are shown in following Table 1 and Table 2.

Table 1 Ingredients of Strontium-doped Root Canal Filling Paste of Examples 1-11

| Serial No. / Component | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| (a) | 35% tricalcium silicate | 27% tricalcium silicate | 35% dicalcium silicate | 35% tricalcium silicate | 35% tricalcium silicate | 35% tricalcium silicate | 35% tricalcium silicate | 35% tricalcium silicate | 35% tricalcium silicate | 35% tricalcium silicate | 35% tricalcium silicate |
| (b) | 2% strontium phosphate | 10% strontium phosphate | 2% strontium phosphate | 2% strontium chloride | 2% strontium nitrate | 2% strontium fluoride | 2% strontium ranelate | 2% strontium acetate | 2% strontium phosphate | 2% strontium phosphate | 2% strontium phosphate |
| (c) | 33% zirconium oxide | 33% zirconium oxide | 33% zirconium oxide | 33% zirconium oxide | 33% zirconium oxide | 33% zirconium oxide | 33% zirconium oxide | 33% zirconium oxide | 33% zirconium oxide | 33% zirconium oxide | 33% zirconium oxide |
| (d) | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate |
| (e) | 25% polyethylene glycol | 25% polyethylene glycol | 25% polyethylene glycol | 25% polyethylene glycol | 25% polyethylene glycol | 25% polyethylene glycol | 25% polyethylene glycol | 25% polyethylene glycol | 25% propylene glycol | 25% glycerol | 25% dimethyl sulfoxide |

Table 2 Ingredients of Strontium-doped Root Canal Filling Paste of Examples 12-16

| Serial No. / Component | Example 12 | Example 13 | Example 14 | Example 15 | Example 16 |
|---|---|---|---|---|---|
| (a) | 40% tricalcium silicate | 20% tricalcium silicate | 25% dicalcium silicate | 30% tricalcium silicate | 20% tricalcium silicate |
| (b) | 0.5% strontium phosphate | 5% strontium acetate | 7% strontium phosphate | 4% strontium chloride | 3% strontium nitrate |
| (c) | 30% columbium monoxide | 40% bismuth trioxide | 35% columbium dioxide | 37% barium sulfate | 33% iodoform |
| (d) | 9.5% tetracalcium phosphate | 10% α-tricalcium phosphate | 5% calcium dihydrogen phosphate | 7% β-tricalcium phosphate | 4% calcium dihydrogen phosphate |
| (e) | 20% polyethylene glycol | 25% polyethylene glycol | 28% polyethylene glycol | 22% polyethylene glycol | 40% polyethylene glycol |

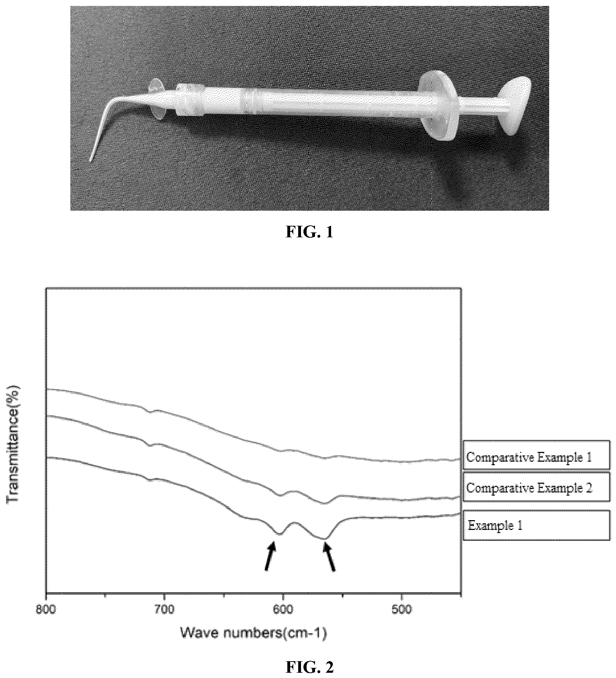

Examples 1-16 further respectively provide a preparation method for a strontium-doped root canal filling paste. The preparation methods of Examples 1-16 are the same, and the difference merely lies in changing ratios of relevant raw materials. Illustration is given by taking Example 1 as an example. Corresponding raw materials were accurately weighed with reference to the ratios provided in Table 1 and Table 2 above, and powders of the component (a) and the component (b) were put into a ball mill and ball-milled uniformly (a media-to-material ratio was 10:1, and a rotational speed was 350 r/min), so as to obtain a strontium-physically-doped calcium silicate mixed powder (strontium calcium silicate for short). Then the strontium calcium silicate, the component (c), the component (d) and the component (e) were put into a mixer and stirred uniformly, so as to obtain a uniformly mixed paste. Finally the mixed paste was dispensed into a medical syringe fitted with an injection hose needle, so as to obtain a syringe containing the strontium-doped root canal filling paste (see FIG. 1). 16 groups of root canal filling paste were prepared in total in Examples 1~16 by the same method.

Comparative Examples 1-5

Comparative Examples 1-5 respectively provide a root canal filling paste, of which ingredients are shown in following Table 3.

## Table 3 Ingredients of Root Canal Filling Paste

| Serial No. / Component | Comparative Example 1 | Comparative Example 2 | Comparative Example 3 | Comparative Example 4 | Comparative Example 5 |
|---|---|---|---|---|---|
| (a) | 37% tricalcium silicate | 37% strontium-doped tricalcium silicate ($Sr_{0.18}Ca_{2.82}SiO_5$) | 22% tricalcium silicate | 42% tricalcium silicate | 10% tricalcium silicate |
| (b) | 0 | 0 | 15% strontium phosphate | 0 | 0 |
| (c) | 33% zirconium oxide | 33% zirconium oxide | 33% zirconium oxide | 33% zirconium oxide | 60% zirconium oxide |
| (d) | 5% calcium dihydrogen phosphate | 5% calcium dihydrogen phosphate | 5% calcium dihydrogen phosphate | 0 | 5% calcium dihydrogen phosphate |
| (e) | 25% polyethylene glycol | 25% polyethylene glycol | 25% polyethylene glycol | 25% polyethylene glycol | 25% polyethylene glycol |

With regard to the preparation method for the root canal filling paste of Comparative Examples 1-11, if strontium compounds were not doped, the root canal filling paste was prepared through following steps: putting the component (a), the component (c), the component (d) and the component (e) in a mixer and stirring them uniformly, so as to obtain a uniformly mixed paste. Finally the mixed paste was dispensed into a medical syringe fitted with an injection hose needle, so as to obtain a syringe containing the root canal filling paste. If strontium compounds were doped, the root canal filling paste was prepared with reference to the preparation method in Example 1, and only types and ratios of the raw materials were changed correspondingly.

Comparative Examples 6-11

Comparative Example 6-11 respectively provide a strontium-doped root canal filling paste, of which ingredients are shown in following Table 4.

Table 4 Ingredients of Strontium-doped Root Canal Filling Paste

| Serial No.<br><br>Component | Comparative Example 6 | Comparative Example 7 | Comparative Example 8 | Comparative Example 9 | Comparative Example 10 | Comparative Example 11 |
|---|---|---|---|---|---|---|
| (a) | 35% tricalcium silicate | 35% tricalcium silicate | 35% tricalcium silicate | 35% tricalcium silicate | 35% tricalcium silicate | 35% tricalcium silicate |
| (b) | 2% strontium glass powder | 2% strontium sulfate | 2% strontium zirconate | 2% strontium aluminate | 2% strontium tungstate | 2% strontium molybdate |
| (c) | 33% zirconium oxide | 33% zirconium oxide | 33% zirconium oxide | 33% zirconium oxide | 33% zirconium oxide | 33% zirconium oxide |
| (d) | 5% calcium dihydrogen phosphate | 5% calcium dihydrogen phosphate | 5% calcium dihydrogen phosphate | 5% calcium dihydrogen phosphate | 5% calcium dihydrogen phosphate | 5% calcium dihydrogen phosphate |
| (e) | 25% polyethylene glycol | 25% polyethylene glycol | 25% polyethylene glycol | 25% polyethylene glycol | 25% polyethylene glycol | 25% polyethylene glycol |

Detection Example 1

According to Pharmaceutical Industry Standard of the People's Republic of China: Dental Root Canal Sealing Materials (YY0717-2009), the properties of the root canal filling pastes prepared in Examples 1~11, Comparative Examples 1-5 and Comparative Examples 6-11 were respectively detected, wherein detection items included appearance, fluidity, and curing time. Test results of the properties are shown in Table 5, Table 6 and Table 7.

Table 5 Detection Results (1)

| Serial No. / Property | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| Appearance | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters |
| Fluidity | 26 mm | 24 mm | 24 mm | 25 mm | 24 mm | 25 mm | 25 mm | 24 mm | 25 mm | 24 mm | 24 mm |
| Curing Time | 2.5 h | 3 h | 3 h | 3 h | 3.5 h | 3.5 h | 3.5 h | 3 h | 3 h | 3.5 h | 3.5 h |

## Table 6 Detection Results (2)

| Serial No. / Property | Comparative Example 1 | Comparative Example 2 | Comparative Example 3 | Comparative Example 4 | Comparative Example 5 |
|---|---|---|---|---|---|
| Appearance | Uniform appearance, | Uniform appearance, | Uniform appearance, | Uniform appearance, | Uniform appearance, |

| | without visible foreign matters | without visible foreign matters | without visible foreign matters | without visible foreign matters | without visible foreign matters |
|---|---|---|---|---|---|
| Fluidity | 25 mm | 25 mm | 26 mm | 28 mm | 18 mm |
| Curing Time | 19 h | 4 h | 8 h | 32 h | 35 h |

Table 7 Detection Results (3)

| Serial No. / Property | Comparative Example 6 | Comparative Example 7 | Comparative Example 8 | Comparative Example 9 | Comparative Example 10 | Comparative Example 11 |
|---|---|---|---|---|---|---|
| Appearance | Uniform appearance, without visible foreign matters | Uniform appearance, without visible foreign matters | Uniform appearance, without visible foreign matters | Uniform appearance, without visible foreign matters | Uniform appearance, without visible foreign matters | Uniform appearance, without visible foreign matters |
| Fluidity | 20 mm | 22 mm | 19 mm | 21 mm | 26 mm | 24 mm |
| Curing Time | 16 h | 14 h | 15 h | 14 h | 17 h | 13 h |

It can be seen from Table 5 and Table 6 that the strontium-doped root canal filling pastes provided in the examples of the present application have a uniform appearance, without visible foreign matters. The fluidities were 24-26 mm, with no significant difference.

By comparing Examples 1 and 2 and Comparative Example 1 and Comparative Example 3, it can be seen that when a strontium doping amount is 0 (namely, without strontium doping), the curing time is 19 h. When 2% or 10% strontium phosphate is doped, the curing time can be greatly shortened. In the above, when the doping amount is 2%, the curing time is the shortest, only 2.5 h. However, when the doping amount is 15%, the curing time is increased to 8 h. It indicates that the strontium doping can significantly shorten the curing time, and it is not the case that a higher doping amount is better, while a high strontium doping amount on the contrary may prolong the curing.

It can be seen by comparing Example 1 and Comparative Example 2 that the physical doping of the strontium compound (strontium phosphate) + tricalcium silicate, compared with chemically doping a single component strontium-doped tricalcium silicate, greatly reduces the curing time of the prepared root canal filling paste.

It can be seen by comparing Comparative Example 1, Comparative Example 4 and Comparative Example 5 that when the content of the tricalcium silicate is reduced to 10% or increased to 42%, the properties are adversely affected.

It can be seen by comparing Example 1 and Examples 9~11 that when a liquid phase is polyethylene glycol, propylene glycol, glycerol, dimethyl sulfoxide, etc., the root canal filling paste can be successfully prepared.

According to Table 7, it can be seen from the comparison between Example 1, Comparative Example 1 and Comparative Examples 6-11 that not all doping of strontium compounds can achieve very good effects. The improvement on the curing time is less for Comparative Examples 6-11.

Detection Example 2

*In vitro* Test for Biological Activity of Strontium-doped Root Canal Filling Paste

Samples of the strontium-doped root canal filling pastes prepared in Example 1, Comparative Example 1 and Comparative Example 2 were compared and tested for remineralization property with reference to an infrared spectroscopy method in T/CSBM 0026-2022 In vitro mineralization test method of bioactive glass. Infrared characteristic absorption peaks of mineralization products of different reactions were analyzed, wherein judgment basis was whether a P-O bending vibration absorption peak representing a crystalline state appeared at 610 cm ~ 600 cm and 560 cm ~ 550 cm in a Fourier transform infrared spectrum, and intensities of mineralization absorption peaks were compared. Infrared spectroscopy test results of the root canal filling pastes after 4 h soaking in SBF are shown in FIG. 2.

According to FIG. 2, it can be seen that infrared curves of all samples have double vibration absorption peaks at 610 cm ~ 600 cm and 560 cm ~ 550 cm (indicated by arrows in FIG. 2), which indicates that all the root canal filling pastes prepared in Example 1, Comparative Example 1 and Comparative Example 2 have biological activity/mineralization characteristic. It can be seen from comparison of peak intensities that the peak intensity of Comparative Example 1 is very weak, indicating that the root canal filling paste without strontium doping has very low biological activity. The peak intensity of Comparative Example 2 is enhanced, indicating that the root canal filling paste prepared by using the strontium-doped tricalcium silicate formed by chemically doping has improved biological activity. The peak intensity of Example 1 is greatly improved, indicating that the root canal filling paste prepared by physically mixing and doping the strontium compound and tricalcium silicate has the best biological activity. This may be due to the way of strontium doping by physical mixing, where strontium elements can be quickly released, thereby realizing true induced remineralization.

Detection Example 3

Detection of the bonding strength of the strontium-doped root canal filling paste with the root canal wall

30 extracted teeth (premaxillary teeth, and single root canal) were collected. The extracted teeth were subjected to root canal preparation through a standard root canal preparation procedure, and were respectively filled with the root canal filling pastes prepared in Example 1, Comparative Example 1 and Comparative Example 2 in cooperation with gutta percha point by single-cone filling.

All the samples were embedded in wax blocks, and cut at intervals of 1 mm in a direction from root to crown by a low-speed slice machine, with a blade being perpendicular to a long axis of teeth. 9 teeth sheets with a thickness of (1.00 $\pm$0.20) mm were obtained from each sample, and a fifth sheet was taken as an experimental sample. Thickness h (accurate to 0.01 mm) of each tooth sheet was measured by a vernier caliper, a crown diameter D1 and a root diameter D2 of each tooth sheet were measured under a microscope for all the sheets, and an adhesive area A was calculated according to a formula:

$$A=0.5\times\pi\times(D1+D2)\times h$$

Each sample tooth sheet was fixed on a fixture dedicated to push-out test by sticky wax, and then was placed on a loading platform of a mechanical testing machine. A matched loading head was chosen. The loading head was adjusted in position so that it is perpendicular to the tooth sheet and only contacts the filling material. A displacement speed of crossbeam was controlled at 0.05 mm/min. Load F when the filling material moved was recorded. Adhesive strength was calculated according to formula P = F/A. Mean values of adhesion strength results are shown in Table 8.

Table 8 Dectection Results

| Group | Bonding Strength |
|---|---|
| Example 1 | 4.06 MPa |
| Comparative Example 1 | 1.98 MPa |
| Comparative Example 2 | 2.85 MPa |

It can be seen from Table 8 that Example 1 and Comparative Example 2 both render higher bonding strength to the root canal wall than Comparative Example 1, which may be due to improved mineralization property of the samples by the strontium doping. It can be seen from comparison between Example 1 and Comparative Example 2 that the bonding strength with the root canal wall in Example 1 is significantly higher than that of Comparative Example 2, which indicates that the root canal filling paste prepared by mixed doping of strontium compound and tricalcium silicate is best bonded with the root canal wall. This may be due to the best mineralizability/biological activity of the root canal filling paste prepared by the strontium doping in a physical mixing manner, and the hydroxyapatite formed by the mineralization penetrates into dentine pores of the root canal wall, so that the bonding strength is higher, and the root canal sealing ability is also better.

**Claims**

1.   A strontium-doped root canal filling paste, **characterized in that** raw materials of the strontium-doped root canal filling paste comprise a component (a) and a component (b), and wherein the component (a) is selected from calcium silicate compounds, the component (b) is selected from strontium compounds, the component (a) accounts for 20%-40% of a total mass of the raw materials, the component (b) accounts for 0.5%-10% of the total mass of the raw materials; and the component (a) and the component (b) are doped in a physical doping manner.

2. The strontium-doped root canal filling paste according to claim 1, wherein the component (a) accounts for 25%-35% of the total mass of the raw materials; and
preferably, the component (a) accounts for 2-5% of the total mass of the raw materials.

3. The strontium-doped root canal filling paste according to claim 1 or 2, wherein the calcium silicate compounds are any one or a combination of any two or more selected from the group consisting of tricalcium silicate, dicalcium silicate and monocalcium silicate; and
preferably, the strontium compound is any one or a combination of any two or more selected from the group consisting of strontium chloride, strontium phosphate, strontium nitrate, strontium fluoride, strontium acetate and strontium ranelate.

4. The strontium-doped root canal filling paste according to any one of claims 1 to 3, wherein the raw materials further comprise a component (c), and the component (c) is selected from radiation inhibitors;

preferably, the radiation inhibitors are selected from the group consisting of metal oxides, metal sulfate compounds and polyhalogenated C1-C3 alkyl;
preferably, the radiation inhibitors are selected from the group consisting of transition metal oxides, metal sulfate compounds and polyiodine-substituted C1-C3-alkyl; and
preferably, the radiation inhibitors are any one or a combination of any two or more selected from the group consisting of niobium oxides, zirconium oxides, bismuth oxides, tantalum oxides, barium sulfate and iodoform.

5. The strontium-doped root canal filling paste according to claim 4, wherein the component (c) accounts for 30%-40% of the total mass of the raw materials, preferably 30%-35%.

6. The strontium-doped root canal filling paste according to any one of claims 1 to 5, wherein the raw materials further comprise a component (d), and the component (d) is selected from calcium phosphate compounds, preferably, any one or a combination of any two or more selected from the group consisting of $\alpha$-tricalcium phosphate, $\beta$-tricalcium phosphate, tetracalcium phosphate, calcium dihydrogen phosphate and hydroxyapatite; and
preferably, the component (d) accounts for 2%-10% of the total mass of the raw materials, preferably 3%-5%.

7. The strontium-doped root canal filling paste according to any one of claims 1 to 6, wherein the raw materials further comprise a component (e), and the component (e) is selected from organic solvents which can be mixed with the component (a) and the component (b) to form a paste;

preferably, the organic solvents are any one or a combination of any two or more selected from the group consisting of alcohol solvents, polymer solvents and sulfoxide solvents;
preferably, the organic solvents are selected from the group consisting of C2-C5 polyol, poly C2-C5 polyol, polypyrrolidine compounds and C2-C5 sulfoxide solvents;
preferably, the organic solvents are any one or a combination of any two or more selected from the group consisting of polyethylene glycol, propylene glycol, glycerol, polyvinylpyrrolidone and dimethyl sulfoxide; and
preferably, the component (e) accounts for 20%-40% of the total mass of the raw materials.

8. Use of the strontium-doped root canal filling paste according to any one of claims 1-7 in preparation of a material for treatment of a dental pulp disease.

9. A preparation method for the strontium-doped root canal filling paste of any one of claims 1-7, comprising: physically mixing the component (a) and the component (b) uniformly.

10. The preparation method according to claim 9, comprising: ball-milling and mixing the component (a) and the component (b) to form a mixed powder; and then mixing the mixed powder with at least one of a component (c), a component (d) and a component (e) uniformly.

**FIG. 1**

**FIG. 2**

Europäisches
Patentamt
European
Patent Office
Office européen
des brevets

**EUROPEAN SEARCH REPORT**

**Application Number**

EP 24 18 1862

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/065946 A1 (SEPTODONT OU SEPTODONT SAS OU SPECIALITES SEPTODONT [FR] ET AL.) 24 May 2012 (2012-05-24) | 1-5,7-10 | INV.<br>A61K6/54<br>A61K6/56 |
| A | * page 12, examples, compositions 2 and 3; page 6, line 6 - page 11, line 28 * | 6 | A61K6/849<br>A61K6/802 |
| | ----- | | A61K6/818 |
| X | CN 112 843 341 A (UNIV ANHUI MEDICAL) 28 May 2021 (2021-05-28) | 1,3,4,<br>6-10 | C01B33/24<br>A61K6/853 |
| A | * example 5 * | 2,5 | |
| | ----- | | |
| A | US 2012/012030 A1 (SAGHIRI MOHAMMAD ALI [IR] ET AL) 19 January 2012 (2012-01-19) * paragraphs [0025] - [0038]; tables 1,2 * | 1-10 | |
| | ----- | | |
| A | WO 01/41821 A1 (BIOSYNTECH CANADA INC [CA]; CHAPUT CYRIL [CA] ET AL.) 14 June 2001 (2001-06-14) * page 22, line 18 - page 25, line 6 * | 1-10 | |
| | ----- | | |

| TECHNICAL FIELDS SEARCHED (IPC) |
|---|
| A61K<br>C01B<br>C04B |

The present search report has been drawn up for all claims

| Place of search | Date of completion of the search | Examiner |
|---|---|---|
| The Hague | 8 February 2025 | Panitz, J |

EPO FORM 1503 03.82 (P04C01)

## ANNEX TO THE EUROPEAN SEARCH REPORT
## ON EUROPEAN PATENT APPLICATION NO.

EP 24 18 1862

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2025

| Patent document cited in search report | | Publication date | Patent family member(s) | | Publication date |
|---|---|---|---|---|---|
| WO 2012065946 | A1 | 24-05-2012 | AR | 084404 A1 | 15-05-2013 |
| | | | AU | 2011307108 A1 | 31-05-2012 |
| | | | BR | 112012012805 A2 | 20-06-2017 |
| | | | CA | 2773421 A1 | 15-05-2012 |
| | | | CN | 102958494 A | 06-03-2013 |
| | | | DK | 2637628 T3 | 30-01-2017 |
| | | | EP | 2452667 A1 | 16-05-2012 |
| | | | EP | 2637628 A1 | 18-09-2013 |
| | | | EP | 2865366 A1 | 29-04-2015 |
| | | | ES | 2609436 T3 | 20-04-2017 |
| | | | JP | 5824518 B2 | 25-11-2015 |
| | | | JP | 2013536831 A | 26-09-2013 |
| | | | JP | 2015231992 A | 24-12-2015 |
| | | | KR | 20130057410 A | 31-05-2013 |
| | | | MX | 351347 B | 11-10-2017 |
| | | | PL | 2637628 T3 | 30-06-2017 |
| | | | TW | 201223548 A | 16-06-2012 |
| | | | US | 2012270184 A1 | 25-10-2012 |
| | | | WO | 2012065946 A1 | 24-05-2012 |
| | | | ZA | 201202422 B | 28-08-2013 |
| CN 112843341 | A | 28-05-2021 | NONE | | |
| US 2012012030 | A1 | 19-01-2012 | NONE | | |
| WO 0141821 | A1 | 14-06-2001 | AT | E243049 T1 | 15-07-2003 |
| | | | AT | E247495 T1 | 15-09-2003 |
| | | | AU | 1848601 A | 18-06-2001 |
| | | | AU | 1979201 A | 18-06-2001 |
| | | | DE | 60003459 T2 | 06-05-2004 |
| | | | DE | 60004710 T2 | 08-07-2004 |
| | | | EP | 1237585 A1 | 11-09-2002 |
| | | | EP | 1255576 A1 | 13-11-2002 |
| | | | US | 2003199615 A1 | 23-10-2003 |
| | | | US | 2010029549 A1 | 04-02-2010 |
| | | | WO | 0141821 A1 | 14-06-2001 |
| | | | WO | 0141822 A1 | 14-06-2001 |

EPO FORM P0459

For more details about this annex : see Official Journal of the European Patent Office, No. 12/82

**REFERENCES CITED IN THE DESCRIPTION**

**Patent documents cited in the description**

- US 5769638 A
- CN 113304056 B